# EUROPEAN PATENT SPECIFICATION

(11) **EP 4 205 844 B1**
(45) Date of publication and mention of the grant of the patent: **09.09.2026**
(21) Application number: 21895091.3
(22) Date of filing: 17.11.2021
(51) Int. Cl.: B01J 23/00, B01J 37/00, B01J 37/08, B01J 37/04, B01J 8/24, C07C 253/24, C07C 255/08

(54) **AMMOXIDATION CATALYST, METHOD FOR PRODUCING SAME, AND METHOD FOR PRODUCING ACRYLONITRILE USING AMMOXIDATION CATALYST**
AMMOXIDATIONSKATALYSATOR, VERFAHREN ZUR HERSTELLUNG DAVON UND VERFAHREN ZUR HERSTELLUNG VON ACRYLNITRIL UNTER VERWENDUNG DES AMMOXIDATIONSKATALYSATORS
CATALYSEUR D'AMMOXYDATION, SON PROCÉDÉ DE PRODUCTION, ET PROCÉDÉ DE PRODUCTION D'ACRYLONITRILE À L'AIDE DU CATALYSEUR D'AMMOXYDATION

(30) Priority: 17.11.2020 KR 20200154030; 16.11.2021 KR 20210157833
(43) Date of publication of application: 05.07.2023
(73) Proprietor: LG Chem, Ltd., Seoul 07336 (KR)
(72) Inventor: RYOU, Youngseok, Daejeon 34122 (KR); CHOI, Jae Hyung, Daejeon 34122 (KR); PARK, Hongseok, Daejeon 34122 (KR); CHOI, Jun Seon, Daejeon 34122 (KR)
(74) Representative: Goddar, Heinz J.
(86) International application number: PCT/KR2021/016853
(87) International publication number: WO 2022/108323

(56) References cited:
- EP-A1- 0 713 724
- JP-A- 2000 344 724
- KR-A- 20050 086 244
- KR-A- 20130 060 184
- KR-A- 20200 010 168
- KR-B1- 101 512 483
- US-A- 4 040 978
- US-A1- 2019 126 262
- US-B1- 6 642 405

## Description

### [TECHNICAL FIELD]

### CROSS-REFERENCE TO RELATED APPLICATION(S)

The present disclosure relates to an ammoxidation catalyst that exhibits excellent activity, reaction conversion rate and selectivity for the ammoxidation reaction of propylene while exhibiting improved stability under a high temperature, and a method for preparing the same, and a method for preparing acrylonitrile using the ammoxidation catalyst.

### [BACKGROUND ART]

The ammoxidation process of propylene refers to a process of preparing acrylonitrile based on a reduction reaction process for reacting ammonia with propylene and a mechanism of reoxidation by oxygen. For such ammoxidation process of propylene, catalysts with various compositions have been studied to increase the conversion rate of the reactants (i.e., propylene), the selectivity and yield of the reaction product (i.e., acrylonitrile).

Specifically, since a composite oxide catalyst containing Mo (molybdenum)-Bi (bismuth) as a main component has been proposed, catalysts to which metals having various oxidation states are added have been studied to enhance their catalytic activity and stability. This type of ammoxidation catalysts is disclosed e.g. in JP 2000 344724 A.

On the other hand, since the ammoxidation reaction is an exothermic reaction that is carried out at a high temperature of 400°C or higher, a recovery of the reaction heat and a temperature control of the reactor are important for the safety and efficiency of the overall reaction process. Thus, in order to ensure heat generation, temperature control, and the like, it is considered that the ammoxidation reaction is mainly carried out in a fluidized bed reactor.

For the ammoxidation reaction in such a fluidized bed reactor, conventionally, the composite oxide catalyst has been mainly prepared in the form of spherical particles through a spray drying method. In such a spray drying method, each precursor solution is mixed and spray-dried to obtain a dried spherical precursor particle, and then the precursor particle is sintered at a high temperature to prepare a composite oxide catalyst.

Therefore, it is very important to appropriately control the conditions such as spray drying and sintering and adjust the particle size distribution, particle strength, and the like of the catalyst particle to a range preferable as a catalyst for a fluidized bed reactor. However, when the composite oxide catalyst particle is prepared through a high-temperature sintering, the catalyst is unstable under a high temperature, and thus, a phenomenon in which molybdenum oxide or the like in the form of fine rods is desorbed from the catalyst is observed in many cases.

When the ammoxidation process is carried out in a high-temperature fluidized bed reactor due to such desorption of the molybdenum component, there is a problem that the structural stability of the catalyst is greatly reduced depending on the usage time, and the activity, yield and/or conversion rate of the catalyst is lowered. Moreover, the desorbed molybdenum component, or the catalyst whose particle size has become smaller due to the desorption passes to a rear end of the reactor, which causes a problem that the catalyst efficiency in the reactor is further reduced. More specifically, this is considered to be because the molybdenum composition of the initial catalyst changes due to the desorbed molybdenum component, and the catalyst whose particle size has become smaller due to desorption passes to a rear end of the reactor and is collected by the cyclone, so that the amount of catalyst in the reactor is substantially reduced. As the actual amount of catalyst in the reactor decreases, the amount of catalyst that needs to be additionally filled for the same reaction efficiency in the reactor increases and the overall reaction efficiency decreases, and the rear end of the reactor may be clogged due to small particles of catalyst or the like.

Previously, in order to solve various problems caused by the desorption of the molybdenum component, attempts have been made to further include various metal components in the composite oxide catalyst and thus improve the thermal and structural stability of the molybdenum-containing composite oxide catalyst. However, there were drawbacks in that not only it was difficult to effectively suppress the desorption of the molybdenum component even by the addition of such a metal component, but also the content of molybdenum, which is an active species of the main catalyst, is reduced by the addition of the various metal components, and thus the overall activity and yield of the catalyst are lowered.

Due to the above-mentioned problems of the prior arts, there is a continuous need to develop an ammoxidation catalyst that exhibits excellent activity and yield for the ammoxidation reaction of propylene, while minimizing the desorption of molybdenum components from the catalyst and exhibiting more improved structural stability under a high temperature.

### [DETAILED DESCRIPTION OF THE INVENTION]

### [Technical Problem]

It is one object of the present disclosure to provide an ammoxidation catalyst that exhibits excellent activity, reaction conversion rate and selectivity for the ammoxidation reaction of propylene while suppressing the desorption of the molybdenum component from the catalyst and thus exhibiting improved structural stability under a high temperature, and a method for preparing the same.

It is another object of the present disclosure to provide a method for preparing acrylonitrile using the ammoxidation catalyst.

### [Technical Solution]

Provided herein is a five-membered ammoxidation catalyst represented by the following Chemical Formula 1:

[Chemical Formula 1] Mo₁₂BiₐNi_{b}Fe_{c}K_{d}Oₓ

in Chemical Formula 1, a to d are molar ratios of each element contained in the catalyst, a is 0.75 to 1.5, b is 5 to 10, c is 1.2 to 3.0, d is 0.01 to 0.5, and x is a numerical value determined according to the oxidation state of each element and the above a to d.

More specifically, a is 0.75 to 1.5, preferably about 0.8 to about 1.2, or about 0.9 to about 1.1, b is 5 to 10, preferably about 6 to about 9, or about 7 to about 8.5, c is 1.2 to 3.0, preferably about 1.5 to about 2.7, or about 1.6 to about 2.5, d is 0.01 to 0.5, preferably about 0.01 to about 0.4, or about 0.01 to about 0.2.

According to an embodiment of the present disclosure, the ammoxidation catalyst may satisfy 1.5 < b/c < 7.5, and more specifically, b/c may be about 2.0 or more, or about 3.0 or more, and about 6.0 or less, or about 5.0 or less, or about 4.5 or less.

Also provided herein is an ammoxidation catalyst composition comprising a carrier, and the ammoxidation catalyst supported on the carrier.

At this time, the carrier includes silica.

Further provided herein is a method for preparing the ammoxidation catalyst, comprising the steps of: forming a precursor solution containing a molybdenum oxide precursor, a bismuth precursor, a nickel precursor, an iron precursor, and a potassium precursor; mixing and spray-drying the precursor solution to prepare a precursor particle; and sintering the precursor particle at a temperature of 500°C or higher.

At this time, the step of forming a precursor solution may include, forming a first precursor solution containing a molybdenum oxide precursor; forming a second precursor solution containing a bismuth precursor, an iron precursor, a cobalt precursor, and a potassium precursor; and mixing and stirring the first and second precursor solutions to form a slurry.

Further, the step of mixing and spray-drying the first and second precursor solutions may include dropwise adding the first precursor solution to the second precursor solution while being stirred; further mixing the first and second precursor solutions under stirring to form a slurry; and spray-drying the slurry through a spraying device having an inlet temperature of 190 to 250°C and an outlet temperature of 110 to 150°C.

Further provided herein is a method for preparing acrylonitrile comprising a step of subjecting propylene and ammonia to ammoxidation reaction in the presence of the above-mentioned catalyst or catalyst composition.

At this time, the reaction step may be carried out at a temperature of 400 to 450°C.

Further, the reaction step may be carried out in a fluidized bed reactor.

As used herein, the terms "a first," "a second," etc. are used herein to explain various constitutional elements, and these terms are used only to distinguish one constitutional element from another constitutional element.

The technical terms used herein is only to explain exemplary embodiments and is not intended to limit the scope of the present disclosure.

The singular forms "a," "an" and "the" are intended to include plural forms, unless the context clearly indicates otherwise.

It should be understood that the terms "comprise," "include", "have", etc. are used herein to specify the presence of stated features, integers, steps, components or combinations thereof, but do not preclude the presence or addition of one or more other features, integers, steps, components, or combinations thereof.

Also, as used herein, in case a layer or an element is mentioned to be formed "on" or "above" layers or elements, it means that the layer or element is directly formed on the layers or elements, or it means that other layers or elements may be additionally formed between the layers, on a subject, or on a substrate.

Now, an ammoxidation catalyst, a method for preparing the same, and a method for preparing acrylonitrile using the same according to specific embodiments of the present disclosure will be described.

According to one embodiment of the present disclosure, there is provided a five-membered ammoxidation catalyst represented by the following Chemical Formula 1:

[Chemical Formula 1] Mo₁₂BiₐNi_{b}Fe_{c}K_{d}Oₓ

in Chemical Formula 1, a to d are molar ratios of each element contained in the catalyst, a is 0.75 to 1.5, b is 5 to 10, c is 1.2 to 3.0, d is 0.01 to 0.5, and x is a numerical value determined according to the oxidation state of each element and the above a to d.

As a result of repeated keen studies by the present inventors, it has been found that in the 5-membered composite oxide catalyst of molybdenum-bismuth-nickel-iron-potassium, when the ratio of each element is adjusted, and in particular, the relative ratio of nickel and iron is adjusted to prepare a five-membered composite oxide catalyst by a spray drying method, it is possible to provide an ammoxidation catalyst in which the desorption of the molybdenum component from the catalyst is suppressed and the structural stability is further improved under a high temperature, thereby completing the present disclosure.

As the ammoxidation catalyst of the one embodiment suppresses the desorption of the molybdenum component at a high temperature, it can greatly reduce the occurrence of problems, such as, for example, the activity, yield and/or conversion rate of the catalyst being reduced by such desorption, or the desorbed component and/or catalyst passing to the rear end of the reactor and thus reducing the catalyst efficiency in the reactor, and the like.

Further, the ammoxidation catalyst of the embodiment can minimize the addition of other metal components due to the structural stability of such a catalyst, and can contain molybdenum, which is a catalytically active species, in a relatively high content ratio. Therefore, excellent activity, yield, reaction conversion rate and selectivity can be achieved in the process of preparing acrylonitrile through ammoxidation reaction of propylene.

On the other hand, in the five-membered catalyst of one embodiment, a to d represent the molar ratio of each remaining metal element contained in the catalyst, based on the molybdenum content ratio of 12, wherein a is 0.75 to 1.5, preferably about 0.8 to about 1.2, or about 0.9 to about 1.1, b is 5 to 10, preferably about 6 to about 9, or about 7 to about 8.5, c is 1.2 to 3.0, preferably about 1.5 to about 2.7, or about 1.6 to about 2.5, and d is 0.01 to 0.5, preferably about 0.01 to about 0.4, or about 0.01 to about 0.2.

Further, x represents the molar ratio of oxygen in the five-membered catalyst, which can be calculated and determined according to the oxidation state of each of the remaining metal elements and the a to d determined above.

When the content ratio of each element in the catalyst of one embodiment is composed in the above-mentioned range, the desorption of the molybdenum component is further minimized, and more excellent activity, yield, reaction conversion rate, selectivity and the like can be exhibited in the ammoxidation reaction of propylene while having excellent high-temperature stability.

Further, when the relative content of molybdenum becomes low due to the addition of other metal components or the like, the content ratio of the catalytically active species is lowered, and the activity of the catalyst or the conversion rate of the ammoxidation reaction may not be sufficient. Conversely, if the relative content ratio of molybdenum is too high, the catalyst of one embodiment does not have sufficient stability, and may cause desorption of large amounts of molybdenum components.

In particular, among the content ratios of the respective elements described above, the ratio of nickel and iron, that is, the relative ratio of b and c, can satisfy 1.5 < b/c < 7.5, and more specifically, b/c may be about 2.0 or more, or about 3.0 or more, and about 6.0 or less, or about 5.0 or less, or about 4.5 or less.

Of the total catalyst composition, nickel and iron serves to re-oxidize the catalyst in a reduced state by using oxygen present in the air during the reaction, Within the above range, nickel and iron form a mixed oxide phase, and such reoxidation efficiency is increased, whereby the overall activity of the catalyst can be further improved.

More specifically, the oxidation number of nickel is usually fixed to be divalent, but the oxidation number of iron can be 2 or 3. Therefore, the form of oxygen vacancy or the oxidation-reduction degree differs depending on the presence ratio of these two atoms, and the difference in the size of the two atoms may cause distortion of the catalyst crystal structure (structural distortion effect). Preferred oxygen vacancy, oxidation-reduction degree, and structural distortion occur within the above ratio range, which can enhance catalytic activity.

The ammoxidation catalyst of the above embodiment may be used in a fluidized bed reactor in the form of a supported catalyst supported on a carrier. The type of the carrier is not particularly limited, and for example, at least one selected from the group consisting of SiO₂, Al₂O₃, MgO, MgCl₂, CaCl₂, ZrO₂, TiO₂, B₂O₃, CaO, ZnO, BaO, ThO₂, SiO₂-Al₂O₃, SiO₂-MgO, SiO₂-TiO₂, SiO₂-V₂O₅, SiO₂-CrO₂O₃, SiO₂-TiO₂-MgO, and zeolite may be used. Among them, a silica (SiO₂)-containing carrier may be typically used.

Further, such a carrier may be contained in an amount of 25 to 75% by weight, or 35 to 65% by weight based on the total weight of the supported catalyst. Thereby, the ammoxidation catalyst of the above one embodiment can be stably supported while not inhibiting its activity.

Meanwhile, the ammoxidation catalyst of the one embodiment described above can be prepared in the form of spherical particles by a spray-drying method, so that it can be preferably used in the ammoxidation reaction of propylene that is carried out at a high temperature of 400°C or higher in a fluidized bed reactor. Thus, according to another embodiment of the present disclosure, there is provided a method for preparing the ammoxidation catalyst of one embodiment by the spray drying method.

The method for preparing such a catalyst may include,
a step of forming a precursor solution containing a molybdenum oxide precursor, a bismuth precursor, a nickel precursor, an iron precursor, and a potassium precursor;
a step of mixing and spray-drying the precursor solution to prepare a precursor particle; and
a step of sintering the precursor particle at a temperature of 500°C or higher.

As described above, each precursor solution is formed and mixed, spray-dried to form a spherical precursor particle, which is then sintered at high temperature, whereby the five-membered composite oxide catalyst of one embodiment may be appropriately prepared in the form of spherical particles suitable for use in a fluidized bed reactor.

According to a more specific embodiment of the preparation method of the other embodiment, the step of forming a precursor solution may include a step of forming a first precursor solution containing a molybdenum oxide precursor; a step of forming a second precursor solution containing a bismuth precursor, an iron precursor, a cobalt precursor, and a potassium precursor; and a step of mixing and stirring the first and second precursor solutions to form a slurry.

Further, according to a more specific embodiment, the step of mixing and spray-drying the first and second precursor solutions may include a step of dropwise adding the first precursor solution to the second precursor solution while being stirred; a step of further mixing the first and second precursor solutions under stirring to form a slurry; and a step of spray-drying the slurry through a spraying device having an inlet temperature of 190 to 250°C and an outlet temperature of 110 to 150°C.

In this manner, a first precursor solution of a molybdenum precursor including the main catalytically active species and a second precursor solution including the remaining metal components are respectively prepared, and then they are added dropwise and further mixed under stirring, and spray-dried. Thereby, a composite oxide catalyst exhibiting more excellent activity can be prepared.

Further, the spray drying step can perform a dry spraying through a spray device having, for example, an inlet temperature of 190 to 250°C, or 200 to 230°C and an outlet temperature of 110 to 150°C, or 120 to 140°C. Thereby, the precursor particles and the catalyst particles prepared therefrom can be obtained in the form of spherical particles having a uniform and desirable particle size distribution. If the inlet temperature during the spray drying is too low, undried fine particles may adhere to the wall surface or lower end of the reactor, making it difficult to obtain catalyst particles having a desired size and shape after drying. Conversely, if the inlet temperature is too high, the particles do not flow sufficiently and dry too quickly, and thus catalyst particles may be formed in an undesirable form such as fine particles or hollow particles. Meanwhile, the outlet temperature of the spraying device can be determined to be a certain level lower than this depending on the inlet temperature.

On the other hand, in the preparation method of the other embodiment described above, as the precursor of each metal element, a precursor of each metal well known from before can be used without particular limitation. Examples of such a precursor include at least one selected from the group consisting of ammonium salts, nitrates, carbonates, chlorides, lactates, hydroxides, organic acid salts, oxides, and hydrates of each metal element.

Further, the precursor of each metal element may be mixed in a molar ratio appropriately selected by those skilled in the art, in consideration of the content ratio of each metal in the catalyst to be finally prepared.

And, after the spray drying, the step of sintering the precursor particles can be carried out, for example, at a temperature of 500°C or higher, or 500°C to 700°C, or 530°C to 600°C for 1 to 6 hours.

On the other hand, except for each of the conditions described above, the catalyst of one embodiment may be prepared according to the method and conditions by a conventional spray drying method, and these specific methods and conditions are also described in Examples described later, and therefore, an additional description thereof will be omitted.

The ammoxidation catalyst of the one embodiment described above may be applied to the ammoxidation reaction of propylene and ammonia typically carried out in a fluidized bed reactor, whereby acrylonitrile can be prepared with high yield, reaction conversion rate and selectivity.

This ammoxidation reaction may be carried out at a temperature of 400 to 450 °C, and may be carried out according to other common methods and conditions of the ammoxidation reaction of propylene.

### [Advantageous Effects]

According to the present disclosure, the content ratio of various metal components can be adjusted, and particularly, the ratio of nickel (Ni) and iron (Fe) can be adjusted to a specific range, thereby providing a molybdenum-containing 5-membered ammoxidation catalyst that can effectively suppress the desorption of the molybdenum component from the catalyst and exhibits more improved structural stability under a high temperature.

Such an ammoxidation catalyst suppresses the desorption of molybdenum oxide in the form of a fine rod, and it may contain molybdenum, which is a catalytically active species, in a relatively high content ratio, while suppressing the problems caused by the desorption of the molybdenum oxide, whereby excellent yield, reaction conversion rate and selectivity can be exhibited in the process of preparing acrylonitrile through ammoxidation of propylene.

### [BRIEF DESCRIPTION OF DRAWINGS]

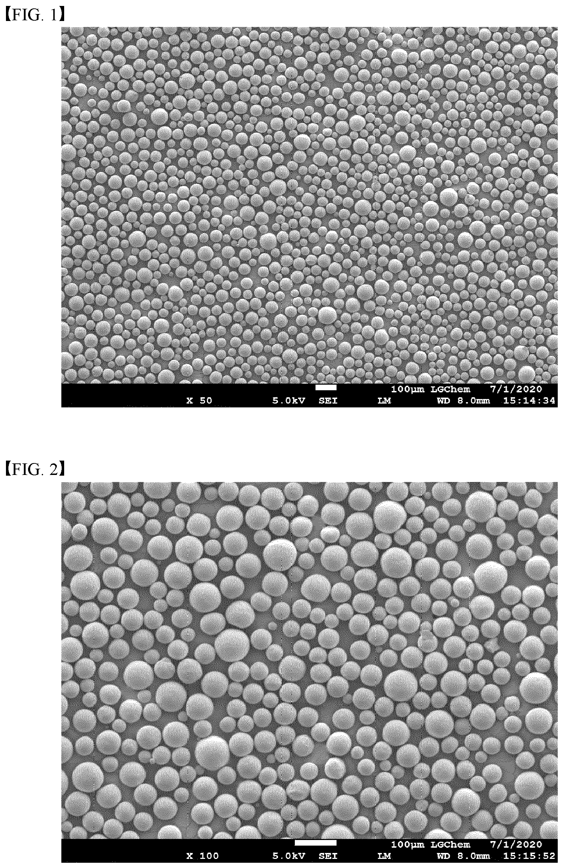

Figs. 1 and 2 are SEM images of precursor particles formed after spray-drying in the process of preparing a catalyst according to an embodiment of the present disclosure.

### [DETAILED DESCRIPTION OF THE EMBODIMENTS]

Hereinafter, the present disclosure will be described in more detail to assist the understanding of the invention. However, the following examples are presented for illustrative purposes only and the contents of the invention is not limited thereby in any way.

### Example: Preparation of Ammoxidation Catalyst

Ammonium paramolybdate [(NH₄)₆Mo₇O₂₄·4H₂O] as a precursor of molybdenum was charged into a reactor, to which hot water at about 100°C was added by 15.8 g to form solution 1. Then, an aqueous oxalic acid solution (a solution in which 1.08 g of oxalic acid was dissolved in 12.44 g of water) was added to 62.5 g of LUDOX AS-40, which is a colloidal silica suspension, and this was added to the solution 1 and stirred for about 30 minutes (formation of a first precursor solution).

A nickel precursor (Ni(NO₃)₂·6H₂O), an iron precursor [Fe(NO₃)₃·9H₂O], and 11.88 g of water were added thereto and stirred well to form a solution. Separately, a bismuth precursor [Bi(NO₃)₃·5H₂O], a potassium precursor [KNO₃], 5.33 g of water, and 1.89 g of nitric acid were added thereto and stirred well to form a solution. These two solutions were mixed and stirred well to form a second precursor solution.

The second precursor solution was added dropwise to the first precursor solution and vigorously stirred. After the addition of the second precursor solution was completed, the mixture was further stirred for 30 minutes to obtain a slurry.

This slurry was spray-dried under conditions of an inlet temperature of 215°C and an outlet temperature of 130 to 140°C to form a precursor particle. The precursor particle was sintered at 580°C for 3 hours in an air atmosphere to prepare a composite oxide catalyst of Example 1.

In Example 1, the precursor particle formed after the spray drying is shown in Figs. 1 and 2, respectively. Referring to Figs. 1 and 2, it can be clearly confirmed that the precursor of the catalyst according to an embodiment of the present disclosure forms intact spherical particles without breaking or cracking under spray-drying conditions.

In each Example and Comparative Example, the amount of the metal precursor used is summarized in Table 1 below.

**[Table 1]**

| Amount of precursor used unit: (g) | Mo | Bi | Ni | Fe | K |
|---|---|---|---|---|---|
| Example 1 | 20.15 | 4.66 | 19.84 | 7.72 | 0.096 |
| Example 2 | 20.03 | 4.63 | 20.28 | 7.68 | 0.096 |
| Example 3 | 19.93 | 4.61 | 20.72 | 7.64 | 0.095 |
| Example 4 | 19.81 | 4.58 | 21.16 | 7.60 | 0.095 |
| Example 5 | 19.71 | 4.56 | 21.59 | 7.55 | 0.094 |
| Example 6 | 19.60 | 4.53 | 22.01 | 7.51 | 0.094 |
| Example 7 | 20.05 | 4.64 | 20.85 | 6.92 | 0.096 |
| Example 8 | 19.81 | 4.58 | 20.60 | 8.35 | 0.095 |
| Example 9 | 19.69 | 4.55 | 20.48 | 9.06 | 0.094 |
| Example 10 | 19.82 | 4.58 | 21.16 | 7.60 | 0.095 |
| Example 11 | 19.70 | 4.56 | 21.03 | 8.31 | 0.094 |
| Example 12 | 19.58 | 4.53 | 20.91 | 9.01 | 0.094 |
| Comparative Example 1 | 22.54 | 5.21 | 13.75 | 4.32 | 0.108 |
| Comparative Example 2 | 20.75 | 4.80 | 12.66 | 8.75 | 0.10 |
| Comparative Example 3 | 19.71 | 4.56 | 20.50 | 3.78 | 0.094 |
| Comparative Example 4 | 18.46 | 4.27 | 19.20 | 11.68 | 0.088 |
| Comparative Example 5 | 18.36 | 4.45 | 19.09 | 12.31 | 0.087 |
| Comparative Example 6 | 18.88 | 4.37 | 19.64 | 7.96 | 0.540 |

### Experimental Example 1: Confirmation of Composition of Catalyst

The composite oxide catalyst prepared in each of Examples and Comparative Examples was analyzed by ICP. An analysis device of ICP-OES (Optima 8300DV, Perkinelmer) was used for such ICP analysis. Further, in this ICP analysis, the specific sample preparation method and analysis conditions were as follows.

First, a sample for analysis was prepared by the following method.

0.2 g of the catalyst sample was placed in a conical tube and weighed accurately. 1 mL of hydrochloric acid and 0.04 mL of hydrofluoric acid were added to this sample, and was quickly sealed. The sample was dissolved in a sonicator for 1 hour, and when the sample was completely dissolved, 1 mL of saturated boric acid water and an internal standard were added, and diluted with 20 mL of ultrapure water. The residual hydrofluoric acid was neutralized at low temperature, shaken and filtered to prepare a sample. ICP analysis was performed on these analysis samples by the following method.

First, ICP-OES analysis equipment was prepared by the following method.
1. Check the condition for the operation of the instrument
2. Gas check: Ar gas, N2 purge gas, Shear gas
3. Operate after checking the power of the instrument cooler
4. Warm up the instrument after checking the instrument communication.

Next, the composition and content of metals (Mo, Bi, Fe, Co, K, Si) included in each catalyst and carrier were analyzed under the following analysis conditions. For reference, the analysis wavelengths for each metal element applied for quantitative analysis of each metal composition are described together at the lower end of the analysis conditions.
* Analysis conditions:
   RF power(W): 1300
   Torch Height(mm): 15.0
   Plasma Gas Flow (L/min): 15.00
   Sample Gas Flow (L/min): 0.8
   Aux. Gas flow (L/min): 0.20
   Pump Speed (mL/min): 1.5
   Internal Standard: Y or Sc
* Analysis wavelength for each metal element: Fe 238.204, Co 228.616, Mo 202.031, Bi 223.061, Si 251.611, K 766.490

By such ICP analysis, the metal composition and content contained in the catalysts produced in Examples and Comparative Examples were elementally analyzed, and the confirmed results are summarized in Table 2 below.

**[Table 2]**

| | Mo | Bi | Ni | Fe | K |
|---|---|---|---|---|---|
| Example 1 | 12 | 1 | 7.1 | 2 | 0.1 |
| Example 2 | 12 | 1 | 7.3 | 2 | 0.1 |
| Example 3 | 12 | 1 | 7.5 | 2 | 0.1 |
| Example 4 | 12 | 1 | 7.7 | 2 | 0.1 |
| Example 5 | 12 | 1 | 7.9 | 2 | 0.1 |
| Example 6 | 12 | 1 | 8.1 | 2 | 0.1 |
| Example 7 | 12 | 1 | 7.5 | 1.8 | 0.1 |
| Example 8 | 12 | 1 | 7.5 | 2.2 | 0.1 |
| Example 9 | 12 | 1 | 7.5 | 2.4 | 0.1 |
| Example 10 | 12 | 1 | 7.7 | 1.8 | 0.1 |
| Example 11 | 12 | 1 | 7.7 | 2.2 | 0.1 |
| Example 12 | 12 | 1 | 7.7 | 2.4 | 0.1 |
| Comparative Example 1 | 12 | 1 | 4.4 | 1 | 0.1 |
| Comparative Example 2 | 12 | 1 | 4.4 | 2.2 | 0.1 |
| Comparative Example 3 | 12 | 1 | 7.5 | 1 | 0.1 |
| Comparative Example 4 | 12 | 1 | 7.5 | 3.3 | 0.1 |
| Comparative Example 5 | 12 | 1 | 7.5 | 3.5 | 0.1 |
| Comparative Example 6 | 12 | 1 | 7.5 | 2.2 | 0.6 |

| | | | | | |
|---|---|---|---|---|---|
| * The molar ratio of oxygen in each catalyst was calculated and determined from the equivalent ratio of each element according to the oxidation state and molar ratio of each of the remaining metal elements. | | | | | |

### Experimental Example 2: Evaluation of Catalyst Performance in Fluidized Bed Reactor

The performances of the catalysts prepared in Examples 1 and 2 and Comparative Example 1 were evaluated in a fluidized bed reactor for preparing acrylonitrile.

Specifically, each reaction gas was supplied to the reactor at a linear velocity of 1 to 7 cm/s, and the composition of such reactive gas was propylene: ammonia: air = 1 : 1.3 : 9.5. The reaction was carried out at 400 to 430°C while maintaining the pressure inside the reactor at normal pressure. In the progress of this reaction, each catalyst of Examples and Comparative Examples was similarly used in an amount of 50 g. HCN contained in the product was passed through a water bath and the ionized cyanide ions were treated with NaOCl or removed through an ion exchange resin.

After the reaction was performed to evaluate the catalyst performance, the reaction product was analyzed using a gas chromatograph (Agilent GC 7890) equipped with a PolaPLOT-Q, Molseive 5A, and chromosorb 101 column.

The initial yield, initial reaction conversion rate and initial reaction selectivity of the catalysts of the respective Examples and Comparative Examples derived from these analysis results are summarized in Table 3.

**[Table 3]**

| | Reaction conversion rate (%) | Reaction selectivity (%) | Yield (%) |
|---|---|---|---|
| Example 1 | 91.7 | 79.7 | 73.1 |
| Example 2 | 94.5 | 80.5 | 76.1 |
| Example 3 | 93.5 | 83.6 | 78.2 |
| Example 4 | 94.5 | 81.3 | 76.8 |
| Example 5 | 91.6 | 81.4 | 74.6 |
| Example 6 | 87.0 | 82.3 | 71.6 |
| Example 7 | 93.5 | 83.6 | 78.2 |
| Example 8 | 96.2 | 84.2 | 81.0 |
| Example 9 | 93.1 | 84.1 | 78.3 |
| Example 10 | 94.5 | 81.3 | 76.8 |
| Example 11 | 96.5 | 81.6 | 78.1 |
| Example 12 | 95.3 | 81.1 | 77.2 |
| Comparative Example 1 | 82.9 | 82.9 | 68.7 |
| Comparative Example 2 | 86.5 | 82.2 | 71.1 |
| Comparative Example 3 | 89.5 | 81.0 | 73.2 |
| Comparative Example 4 | 96.1 | 79.3 | 76.2 |
| Comparative Example 5 | 96.2 | 78.5 | 74.7 |
| Comparative Example 6 | 81.0 | 78.0 | 63.2 |

Referring to Table 3 above, it can be clearly confirmed that the catalysts of Examples exhibit excellent yield, reaction conversion rate and reaction selectivity as compared to Comparative Examples.

## Claims

1. A five-membered ammoxidation catalyst represented by the following Chemical Formula 1:
[Chemical Formula 1] Mo₁₂BiₐNi_{b}Fe_{c}K_{d}Oₓ
in Chemical Formula 1, a to d are molar ratios of each element contained in the catalyst,
a is 0.75 to 1.5,
b is 5 to 10,
c is 1.2 to 3.0,
d is 0.01 to 0.5, and
x is a numerical value determined according to the oxidation state of each element and the above a to d.

2. The ammoxidation catalyst according to claim 1, wherein 1.5 < b/c < 7.5.

3. An ammoxidation catalyst composition comprising a carrier, and the ammoxidation catalyst of claim 1 supported on the carrier.

4. The ammoxidation catalyst composition according to claim 3, wherein the carrier comprises silica.

5. A method for preparing the ammoxidation catalyst of claim 1, comprising the steps of:
forming a precursor solution containing a molybdenum oxide precursor, a bismuth precursor, a nickel precursor, an iron precursor, and a potassium precursor;
mixing and spray-drying the precursor solution to prepare a precursor particle; and
sintering the precursor particle at a temperature of 500°C or higher.

6. The method for preparing the ammoxidation catalyst according to claim 5, wherein the step of forming a precursor solution comprises,
forming a first precursor solution containing a molybdenum oxide precursor;
forming a second precursor solution containing a bismuth precursor, an iron precursor, a nickel precursor, and a potassium precursor; and
mixing and stirring the first and second precursor solutions to form a slurry.

7. The method for preparing the ammoxidation catalyst according to claim 6, wherein the step of mixing and spray-drying the first and second precursor solutions comprises,
dropwise adding the first precursor solution to the second precursor solution while being stirred;
further mixing the first and second precursor solutions under stirring to form a slurry; and
spray-drying the slurry through a spraying device having an inlet temperature of 190 to 250°C and an outlet temperature of 110 to 150°C.

8. A method for preparing acrylonitrile comprising a step of subjecting propylene and ammonia to ammoxidation reaction in the presence of the catalyst of claim 1 or the catalyst composition of claim 3.

9. The method for preparing acrylonitrile according to claim 8, wherein the reaction step is carried out at a temperature of 400 to 450°C.

10. The method for preparing acrylonitrile according to claim 8, wherein the reaction step is carried out in a fluidized bed reactor.

## Patentansprüche

1. Fünfteiliger Ammoxidationskatalysator, dargestellt durch die folgende chemische Formel 1:
[Chemische Formel 1] Mo₁₂BiₐNi_{b}Fe_{c}K_{d}Oₓ
in der chemischen Formel 1 sind a bis d Molverhältnisse von jedem Element, das in dem Katalysator enthalten ist,
a beträgt 0,75 bis 1,5,
b beträgt 5 bis 10,
c beträgt 1,2 bis 3,0,
d beträgt 0,01 bis 0,5, und
x ist ein numerischer Wert, der gemäß dem Oxidationszustand von jedem Element und den obigen a bis d bestimmt wird.

2. Ammoxidationskatalysator nach Anspruch 1, bei dem 1,5 < b/c < 7,5 gilt.

3. Ammoxidationskatalysatorzusammensetzung, umfassend einen Träger und den auf dem Träger getragenen Ammoxidationskatalysator nach Anspruch 1.

4. Ammoxidationskatalysatorzusammensetzung nach Anspruch 3, bei der der Träger Siliciumdioxid umfasst.

5. Verfahren zur Herstellung des Ammoxidationskatalysators nach Anspruch 1, umfassend die Schritte:
Bilden einer Vorläuferlösung, die einen Molybdänoxidvorläufer, einen Bismutvorläufer, einen Nickelvorläufer, einen Eisenvorläufer und einen Kaliumvorläufer enthält;
Mischen und Sprühtrocknen der Vorläuferlösung, um ein Vorläuferteilchen herzustellen; und
Sintern des Vorläuferteilchens bei einer Temperatur von 500°C oder höher.

6. Verfahren zur Herstellung des Ammoxidationskatalysators nach Anspruch 5, bei dem der Schritt des Bildens einer Vorläuferlösung umfasst:
Bilden einer ersten Vorläuferlösung, die einen Molybdänoxidvorläufer enthält;
Bilden einer zweiten Vorläuferlösung, die einen Bismutvorläufer, einen Eisenvorläufer, einen Nickelvorläufer und einen Kaliumvorläufer enthält; und
Mischen und Rühren der ersten und der zweiten Vorläuferlösung, um eine Aufschlämmung zu bilden.

7. Verfahren zur Herstellung des Ammoxidationskatalysators nach Anspruch 6, bei dem der Schritt des Mischens und Sprühtrocknens der ersten und der zweiten Vorläuferlösung umfasst:
tropfenweises Zugeben der ersten Vorläuferlösung zu der zweiten Vorläuferlösung, während sie gerührt wird;
weiteres Mischen der ersten und der zweiten Vorläuferlösung unter Rühren, um eine Aufschlämmung zu bilden; und
Sprühtrocknen der Aufschlämmung durch eine Sprühvorrichtung mit einer Einlasstemperatur von 190 bis 250°C und einer Auslasstemperatur von 110 bis 150°C.

8. Verfahren zur Herstellung von Acrylnitril, umfassend einen Schritt des Unterziehens von Propylen und Ammoniak einer Ammoxidationsreaktion in Gegenwart des Katalysators nach Anspruch 1 oder der Katalysatorzusammensetzung nach Anspruch 3.

9. Verfahren zur Herstellung von Acrylnitril nach Anspruch 8, bei dem der Reaktionsschritt bei einer Temperatur von 400 bis 450°C durchgeführt wird.

10. Verfahren zur Herstellung von Acrylnitril nach Anspruch 8, bei dem der Reaktionsschritt in einem Wirbelschichtreaktor durchgeführt wird.

## Revendications

1. Catalyseur d'ammoxydation à cinq membres représenté par la Formule chimique 1 ci-après :
[Formule chimique 1] Mo₁₂BiₐNi_{b}Fe_{c}K_{d}Oₓ
dans la Formule chimique 1, a à d sont des rapports molaires de chaque élément contenu dans le catalyseur,
a est 0,75 à 1,5,
b est 5 à 10,
c est 1,2 à 3.0,
d est 0,01 à 0,5, et
x est une valeur numérique déterminée en fonction de l'état d'oxydation de chaque élément et de a à d ci-dessus.

2. Catalyseur d'ammoxydation selon la revendication 1, dans lequel 1,5 < b/c < 7,5.

3. Composition de catalyseur d'ammoxydation comprenant un support, et le catalyseur d'ammoxydation selon la revendication 1 soutenu sur le support.

4. Composition de catalyseur d'ammoxydation selon la revendication 3, dans laquelle le support comprend la silice.

5. Procédé de préparation du catalyseur d'ammoxydation selon la revendication 1, comprenant les étapes consistant à :
former une solution de précurseur contenant un précurseur d'oxyde de molybdène, un précurseur de bismuth, un précurseur de nickel, un précurseur de fer et un précurseur de potassium ;
mélanger et sécher par pulvérisation la solution de précurseur pour préparer une particule de précurseur ; et
fritter la particule de précurseur à une température égale ou supérieure à 500°C.

6. Procédé de préparation du catalyseur d'ammoxydation selon la revendication 5, dans lequel l'étape de formation d'une solution de précurseur consiste à :
former une première solution de précurseur contenant un précurseur d'oxyde de molybdène ;
former une deuxième solution de précurseur contenant un précurseur de bismuth, un précurseur de nickel, un précurseur de fer et un précurseur de potassium ; et
mélanger et sécher les première et deuxième solutions de précurseur pour former une suspension.

7. Procédé de préparation du catalyseur d'ammoxydation selon la revendication 6, dans lequel l'étape de mélangeage et de séchage par pulvérisation des première et deuxième solutions de précurseur consiste à :
ajouter goutte-à-goutte la première solution de précurseur à la deuxième solution de précurseur en agitant ;
mélanger encore les première et deuxième solutions de précurseur par agitation pour former une suspension ; et
sécher par pulvérisation la suspension à travers un dispositif de pulvérisation ayant une température d'entrée de 190 à 250°C et une température de sortie de 110 à 150°C.

8. Procédé de préparation de l'acrylonitrile comprenant une étape de soumission du propylène et de l'ammoniac à une réaction d'ammoxydation en présence du catalyseur selon la revendication 1 ou de la composition de catalyseur selon la revendication 3.

9. Procédé de préparation de l'acrylonitrile selon la revendication 8, dans lequel l'étape de réaction est effectuée à une température de 400 à 450°C.

10. Procédé de préparation de l'acrylonitrile selon la revendication 8, dans lequel l'étape de réaction est effectuée dans un réacteur à lit fluidisé.
